# NEUE EUROPÄISCHE PATENTSCHRIFT

(11) **EP 0 539 537 B2**
(45) Veröffentlichungstag und Bekanntmachung des Hinweises auf die Entscheidung über den Einspruch: **27.12.2000**
(45) Hinweis auf die Patenterteilung: 11.09.1996
(21) Anmeldenummer: 92908218.8
(22) Anmeldetag: 23.04.1992
(51) Int. Cl.: G01N 33/10, G01N 21/35

(54) **VERFAHREN UND VORRICHTUNG ZUR IN-LINE NIR-MESSUNG VON SCHÜTTFÄHIGEN NAHRUNGSMITTELN**
PROCESS AND DEVICE FOR THE IN-LINE NIR MEASUREMENT OF POURABLE FOODSTUFFS
PROCEDE ET DISPOSITIF DE MESURE NIR EN LIGNE DE PRODUITS ALIMENTAIRES COULANTS

(30) Priorität: 23.04.1991 SE 9101220
(43) Veröffentlichungstag der Anmeldung: 05.05.1993
(73) Patentinhaber: Bühler AG, 9240 Uzwil (CH); Perten, Peter, 6055 Alpnach (CH)
(72) Erfinder: PERTEN, Peter, CH-6055 Alpnach (CH); TOBLER, Hans, CH-9249 Algetshausen (CH)
(86) Internationale Anmeldenummer: CH9200080
(87) Internationale Veröffentlichungsnummer: WO9218864

(56) Entgegenhaltungen:
- EP-A- 0 250 070
- EP-A- 0 304 232
- EP-A- 0 388 082
- EP-A- 0 404 652
- WO-A-85/00656
- WO-A-85/04957
- DE-A- 3 601 931
- US-A- 4 236 076
- US-A- 4 422 760
- Getreide Mehl und Brot, Band 38, Seiten 3-5 (1984)
- Brockhaus Naturwissenschaft und Technik, Sonderausgabe 1989, Band 4,Seite 33 und Band 5, Seiten 20-21
- ZEISS, Sonderdruck aus INCOM-Band 1990, verteilt von Carl Zeiss, Oberkochen

## Beschreibung

### Technisches Gebiet

Die Erfindung betrifft ein Verfahren zum in-line NIR-Messen von Inhaltsstoffen von Schüttgütern insbesondere von schüttfähigen Nahrungsmittel-Produkten.

### Stand der Technik

Es gibt hierfür eine ganze Anzahl verschiedener Messmethoden, so zum Beispiel NMR-Messtechnik, Mikrowellen- oder kapazitive Methoden. In der Praxis haben nur wenige eine grössere Verbreitung gefunden, teils nur für die Messung eines einzigen Inhaltsstoffes.

Bekannt ist die Messung der Produktfeuchtigkeit mittels Mikrowellen von ganzen Getreidekörnern. Viel verbreiteter ist jedoch in der Getreideverarbeitung die kapazitive Messung wie zum Beispiel in der DOS 3024 794 der Anmelderin beschrieben ist. Die Feststellung des Wassergehaltes am Kanzkorn und auch die Regelung der Wassermenge für die Netzung auf einen bestimmten Wassergehalt kann mit dieser Lösung mit hoher Genauigkeit durchgeführt werden.

Die kapazitive Messung erfolgt über einen Plattenkondensator also über ein grossflächiges Element. Mit Vorteil wird hierfür die Messprobe an den Kondensatorplatten vorbeibewegt, was für eine in-line Messung, also für eine Messung in dem Produktionenfluss ideal ist. Durch entsprechende Ausbildung der Messstrecke können Proben-Mittelwerte gebildet werden.

Für die on-line Messung der Feuchtigkeit von Mehl und Griess mit NIR hat sich interessanterweise eine gegensätzliche Probenaufbereitung aufgezwungen.

Die NIR-Messung bedingt nicht nur eine Lichtquelle, sondern auch eine Optik, welche sinngemäss in der Art einer Punktuellen-Messung funktioniert. Für die Optik schafft bekanntlich jede Bewegung, sei es der Optik oder der Messprobe besondere Probleme und kann ein Unscharfwerden der Oberflächenbeschaffenheit verursachen.

Dieses Problem wird dadurch erfolgreich behoben, indem die Produktprobe für die NIR-Messung nicht nur in konstante Bedingungen zum Beispiel bezüglich Druck und Dichte gebracht wird , sondern für die eigentliche Messung wie für eine Labormessung gestoppt resp. stillgesetzt wird. Mit beachtlichem Erfolg konnte die Anmelderin eine entsprechende Messeinrichtung (siehe EPPS Nr. 179 108) in der Praxis einführen. Als grosser Nachteil wurde jedoch empfunden, dass damit eine Ganzkorn-Messung zum Beispiel der 'Proteingehalt von ganzen Getreidekörnern nicht möglich ist. In der Praxis ergab sich daraus eine nicht besonders angenehme Situation:
- der Wassergehalt des Ganzkornes wird mit kapazitivoder allenfalls Mikrowellen-Messtechnik ermittelt,
- der Proteingehalt von Mehl wird mit der geschilderten NIR-Methode gemessen,
- der Proteingehalt ganzer Körner muss aber im Labor festgestellt werden.

Darüberhinaus gibt es bis heute für Mühlenzwischenprodukte viele On-line-Spezialgeräte, z.B. für die Produktfarbe. In jedem Fall wird eine Kalibrierung mit einem Eichmuster vorgenommen, um alle störenden Parameter ausschalten zu können.

In der WO 85/04957 ist ein Verfahren zur kontinuierlichen, quantitativen Messung von Inhaltsstoffen wie Protein oder Wasser in mehlförmigen Mahlgütern beschrieben, wobei die Mahlgüter gepresst und geglättet werden und anschliessend über eine Messstrecke geleitet werden. Im Bereich eines Messaufnehmers erfolgt eine Verdichtung und Infrarotbestrahlung des Mahlgutes. Mittels des Messaufnehmers werden die reflektierten Strahlen ermittelt.

Während einer Messphase werden mehrere Messmengen für jeden Spektralbereich ausgeführt und ein Mittelwert gebildet, der wiederum mit einem vorgegebenen Sollwert verglichen wird.

Als ganz besonders störend wirkt sich aber die Tatsache aus, dass in ein und demselben Fabrikationsbetrieb, wie dies eine Mühle ist, 3 bis 5 grundverschiedene Wellenlängenbereiche von γ-Strahlen bis zu Mikrowellen eingesetzt werden und so vielfach die Messergebnisse nur sehr schwierig vergleichbar sind, dies obwohl das Produkt anfänglich ganze Körner, später Griess- oder Mehl, grundsätzlich das gleiche ist.

Die Erfindung hat sich nun die Aufgabe gestellt, zumindest einen wesentlichen Teil der geschilderten Nachteile zu beheben und eine In-line-Messung ohne das Erfordernis der Probenherausnahme aus dem Produktionsablauf zu ermöglichen.

### Darstellung der Erfindung

Die erfindungsgemässe Lösung ist dadurch gekennzeichnet, dass
- das Produkt als dichter Strom Kontinuierlich vertikal vor einem NIR-Messwertaufnehmer durchgeführt wird
- wobei der, bzw. die Wellenlängenbereiche des reflektierten Lichtes aus einer grösseren Zahl zeitlich nacheinander durchgeführten Einzelmessungen für je ein ganzes Spektrum von einem bewegten Probenbereich, für ein Spektrum unter 100, vorzugsweise mit Messzeiten unter 50 Millisekunden, erfasst wird
- und wobei die Messzeit einer Einzelmessung und die Bewegungsgeschwindigkeit des Produktes aufeinander abgestimmt werden, derart, dass das Produkt sich während der Zeit einer Einzelmessung um weniger als 1/10 der länge des Produktes bewegt,
- und die Inhaltsstoffe über statistische Mittelbildung der relevanten Messwerte ermittelt und auf Grund eines Eichwertes errechnet werden.

Es konnten bereits mit einer Versuchseinrichtung Messwerte mit hoher Genauigkeit ermittelt werden, was insofern überraschte, als sich die Erfindung von der traditionellen Labordenkweise: Präparieren der Probe, Stillsetzten der Probe, allenfalls Aufnahme mehrerer Messwerte der selben Probe und deren Mittelung, völlig löste. Es wird lediglich ein dichter, homogener Strom gefordert und damit eine reproduzierbare Probenoberflächen-Beschaffenheit. Von der Probe selbst wird gefordert, dass sie relativ zu dem Messaufnehmer bewegt wird, bevorzugt vorwärts im Sinne des Produktionsflusses. Es wird eine grosse Zahl Einzelmessungen durchgeführt, dies nun aber von ständig sich änderndem Probenmaterial. Es werden damit bewusst unterschiedliche Messresultate gewonnen.

Wichtig ist dabei, dass eine Vielzahl von Einzelmessungen mit Messzeiten unter 50 Millisekunden gemacht werden, sodass einer oder gegebenenfalls mehrere Inhaltsstoffe entsprechend der ausgewählten Wellenlängenbereich bzw. Bereichen über eine statistische Mittelwertbildung errechnet werden können.

Es wird so doppelt gemittelt, eine Mittelung über die Zeit wie auch über verschiedene Musterbereiche. Trotz der Bewegung des Produktes und der nur sehr kurzen Belichtungszeit können Messwerte von besserer Qualität gegenüber den bisherigen Methoden gewonnen werden.

Ganz besonders überraschend ist aber die Tatsache, dass auch bei der Ganzkornmessung Werte bezüglich der Inhaltsstoffe von hoher Genauigkeit gewonnen werden konnten. Damit ist es aber erstmals möglich geworden, In-line verschiedenartige Inhaltsstoffe wie Wasser, Eiweiss, Asche sowie die Produktfarbe mit dem gleichen System zu ermitteln, sowohl von mehlartigen wie auch körnigen Produkten. Damit aber öffnet sich die Möglichkeit Inhaltsstoffe in einer Mühle von der Einlagerung der ganzen Körner über die Zwischenprodukte bis zu den Fertigprodukten mit ein und demselben Messprinzip zu messen, sodass auch ohne Schwierigkeit sofort vergleich bare Werte entstehen. Das erleichtert sehr stark die Mühlenführung und gestattet mehr als im Stand der Technik die Produktion mit entsprechenden Automatikmitteln zu unterstützen.

Die Bewegung des Produktes vor dem NIR-Messwertaufnehmer wird stetig beziehungsweise konstant gehalten. Bei einzelnen Produktsorten wird vorgeschlagen, die Messzeit einer Einzelmessung und die Bewegungsgeschwindigkeit des Produktes aufeinander abzustimmen derart, dass vorzugsweise das Produkt (im Falle von Getreidekörnern ein Einzelkorn) sich vorzugsweise während der Zeit einer Einzelmessung um wengier als 1/10 der Kornlänge bewegt.

Die neue Erfindung gestattet eine ganze Anzahl besonders vorteilhafter weiterer Ausgestaltungen.

Vorallem bei Produktsorten, die eine beachtliche Inhomogenität aufweisen, ganz besonders für Regelfunktionen zum Beispiel zur Beeinflussung der Anteile einzelner Inhaltsstoffe, hat sich die Erfindung eine vom praktischen Fachmann angewendete Prüfmethode zu eigen gemacht. Diese besteht darin, dass das zu prüfende Material in Händen gehalten, gleichsam von vielen Seiten betrachtet und bewertet wird, in dem das Produkt während der Betrachtung bewegt wird, und man das Muster oft genug sieht. Das neue Verfahren erlaubt zudem in ganz besonders vorteilhafter Weise einander überlappende Probenbereiche zu messen. Wichigt ist aber in jedem Fall, dass eine grose Zahl Messungen vorgenommen werden. Als Erfahrungswert hat sich bisher ergeben,dass die Anzahl der Einzelmessungen mehr als 30 sein sollte.

Gemäss einer weiteren ganz besonders vorteilhaften Ausgestaltung werden alle gewünschten NIR-Wellenlängenbereiche gleichzeitig gemessen, vorzugsweise mit dem Dioden-array-Prinzip. Es werden mit diesem Vorschlag die Messwertegenauigkeit erhöht, insbesondere aber können für die Messungen die Haupt-Elemente der Messeinrichtung unbewegt bleiben. Es sind im wesentlichen keine bewegten Teile notwendig.

Bei einer weiteren Ausführung werden kurzfristig nacheinander einzelne Wellenlängenbereiche gemessen und statistische Auswertungsmethoden verarbeitet.

Ein weiterer bevorzugter Ausbildungsgedanke liegt darin, dass ein kontinuierlicher Produktstrom rückgestaut und im Rückstau gegenüber dem Messwertaufnehmer bewegt wird.

Das was bei der bekannten Anwendung der NIR-Messtechnik schädlich war, wird nun umkehrt als Vorteil genutzt, indem das Produkt bevorzugt vor dem Messwertaufnehmer bewegt wird, beziehungsweise durchläuft.

In einem Mühlenbetrieb ist das Produkt für die Verarbeitung ständig in Bewegung, was nun als Grundlage für die Messung dient indem über statistische Rechenmethoden eine enorme Zahl von wenigstens 10 - 15 bevorzugt aber mehr als 30 Einzelmessungen sowohl von wechselnden Probennahmen wie über die Zeit erfasst und daraus die gewünschten Mittel-Werte errechnet wird. Dies gestattet bei Abweichungen gegenüber einem Sollwert, sofort im Produktfluss oder bei einzelnen Verarbeitungsstufen einzugreifen, da die Fehlwerte innerhalb Sekunden festgestellt werden.

Der Produktrückstau kann in einer Haupt- oder einer By-Passleitung bevorzugt aber über eine Dosierschnecke mit geregelter Durchstromgeschwindigkeit erzeugt werden. Der Rückstau kann gleichzeitig zur Erfassung der Produktdurchsatzes ausgenutzt werden.

Die neue Erfindung betrifft ferner eine Messvorrichtung zum kontinuierlichen Erfassen von Inhaltsstoffen an einem Produkt, insbesondere an einem schüttfähigen Nahrungsmittel mit einer NIR-Messeinrichtung, und ist dadurch gekenntzeichnet, dass sie eine Durchlaufmessstrecke, eine NIR-Messaufnahme-Einrichtung, vorzugsweise für Messserien und jeweiliger Messdauer von unter 100, vorzugsweise 80 unter 50 Millisekunden sowie einen Rechner für die statistische Errechnung von Mittelwerten der jeweiligen Inhaltsstoffe und einen Meßkanal mit Mitteln zur gesteuerten Zwangsaustragsbewegung der Meßprobe aufweist. Es wird ein Dioden-array für die gleichzeitige Erfassung einzelner Spektralbereiche verwendet.

### Kurze Beschreibung der Erfindung

Die Erfindung wird nun mit weiteren Einzelheiten erläutert.

Es zeigen:
- die Figur 1: schematisch einen vollständigen Messaufnehmer mit Verarbeitung der Messwerte
- die Figur 2: den Messaufnehmer in vergrössertem Massstab
- die Figur 3: ein an sich bekanntes NIR-Absorptionsspekturm
- die Figur 4: stellt figürlich eine industriell unbrauchbare Messung eines willkürlichen Produktlaufes
- die Figur 5: ein Messprinzip des Standes der Technik gemäss EPPS Nr. 179 108
- die Figur 6: zeigt eine ganze Messstrecke welche sowohl für Ganzkornmessung wie auch für schwerfliessfähige Produkte geeignet ist
- die Figur 7: das an sich bekannte Dioden-array-Messprinzip schematisch dargestellt.

### Wege zur Ausführung der Erfindung

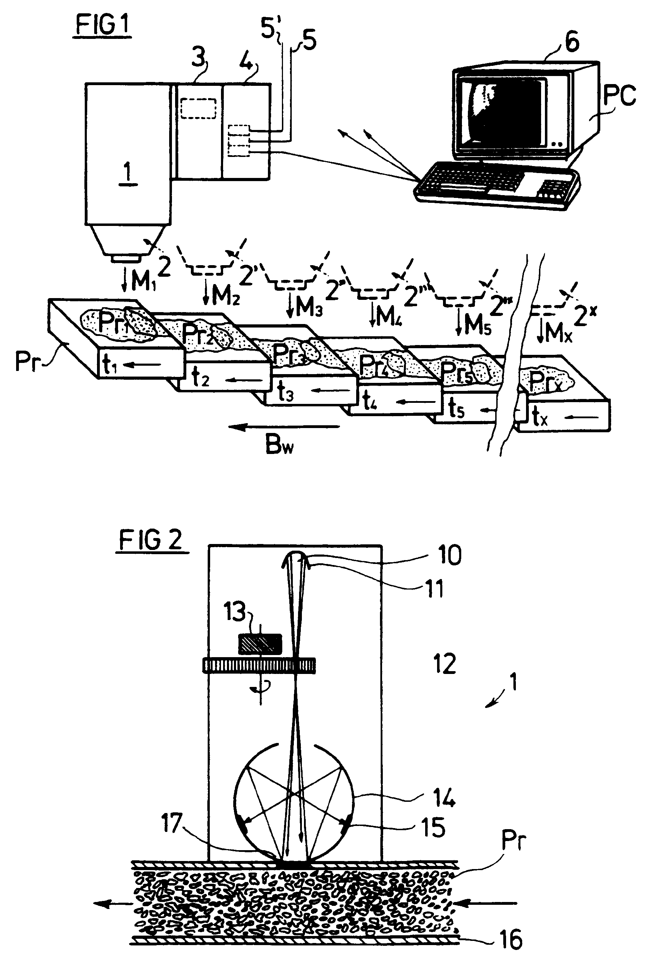

In der Folge wird nun auf die Figur 1 Bezug genommen. Ein Messwertaufnehmer 1 mit Optik mit einem Messkopf 2 ist direkt über einer Probe Pr angeordnet.

An dem Messwertaufnehmer 1 ist eine Vorortelektronik 3 mit einer Wandlereinheit für die Weitergabe von Signalen 5, 5', angeschlossen, und ist verbunden mit einer Rechnereinheit PC, 6. Die Figur 1 soll ein Bewegungsbild darstellen, indem das Produkt stetig von rechts nach links bewegt wird gemäss Pfeil Bw.

Der Messkopf 2,2',2'', 2''',2'^{V},2x ist mehrfach, jedoch strichliert, über je einer anderen Produktpartie Pr₁,Pr₂,Pr₃,Pr₄,Pr₅,Prₓ eingezeichnet. In Wirklichkeit verschiebt sich die Probe Pr vor dem stillstehenden Messkopf 2 und es wird zu dem Zeitpunkt t₁,t₂,t₃,t₄,t₅,tₓ, je eine Messung M₁,M₂,M₃, M₄,M₅,Mₓ, aufgenommen. Wichtig ist, dass die erforderliche Zeit für die Einzelmessung also für M₁ für M₂ usw. in der kleinst möglichen Zeit durchgeführt wird dies im Sinne einer "Blitzlichtaufnahme". Die einzelnen Blitzlichtaufnahmen M₁,M₂,M₃,M₄,M₅,Mₓ werden über die Optik der Vorortelektronik und an den Rechner PC übergeben zur statischen Mittelwertbildung der Messserie zum Beispiel über einen Zeitraum von unter 10 Sekunden.Die Anzahl der Messungen pro Mittelwertbildung kann dabei je nach Aufgabenstellung von mehreren Duzend bis zu hunderten liegen.

Unter dem Begriff "on-line" wird das Messen im Bereich der eigentlichen Produktion verstanden. "On-line" lässt es noch offen, ob die Probe aus dem Fabrikationselement vollständig herausgenommen und ausserhalb in dem Messapparat geprüft wird.

"In-line" besagt präzise, dass das Produkt in der Verarbeitungsmaschine oder Förderleitungen selbst oder in einem aktiv geführten By-Bass, gemessen wird. Bei "In-line" findet keine Probenentnahme, höchstens eine Probeumlenkung und wieder Rückführung statt. Bekannt ist die generelle Problematik. Bei Musterentnahmen ist die Frage der Repräsentanz des Musters beim Messen "In-line", der Ort der Probenmessung besonders wichtig.

In der Figur 2 ist eine NIR-Optik in vergrössertem Massstab dargestellt, dabei wird mit 10 eine Lichtquelle bezeichnet. Ferner weist der Messwertaufnehmer 1 schematisch einen ellepsoidischen Spiegel (Reflektor) 11, ein Filterrad 12 mit Motorantrieb 13, ferner eine Ulbricht'sche Kugel 14, Lichtempfänger 15 sowie eine Produktprobe Pr, welche von dem Messaufnehmer 1 durch ein Fenster 17 getrennt ist und mit einer Referenz verglichen wird, im Sinne einer Kalibrierung. Wesentlich ist, dass das Filterrad mit relativ hoher Geschwindigkeit von mehr als 10 vorzugsweise mehr als 25 Umdrehungen pro Sekunde dreht und eine grosse Zahl von Filtern in Umfangsrichtung zum Beispiel 6, 12 oder 24 aufweist durch welche je das ganze gesuchte Lichtspektrum erfasst wird.

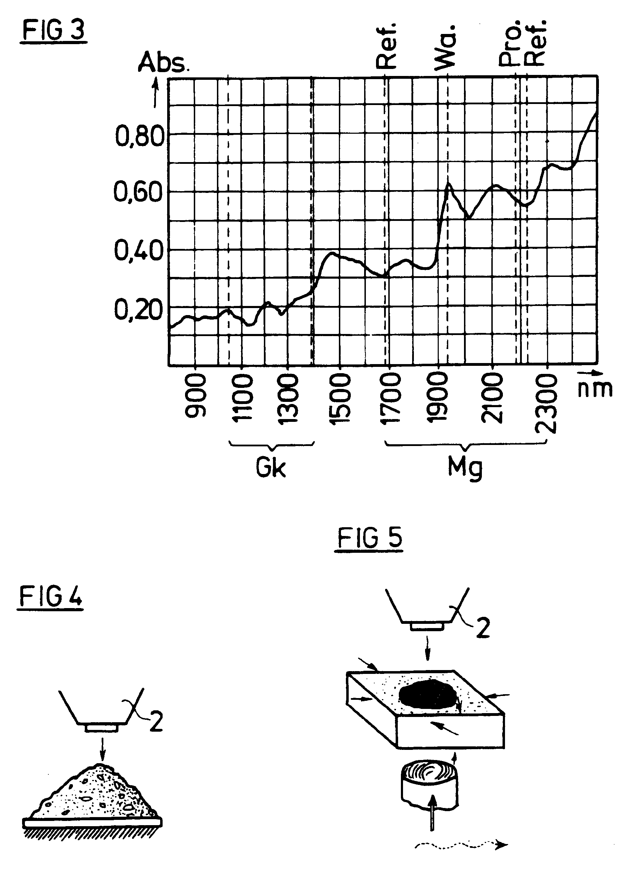

Die Figur 3 zeigt ein bekanntes Absorptionsspektrum von einem mehlartigen Gut. Eigetragen sind für Wasser und Protein je das Wertepaar, wobei die Werte für Wasser über 1900 nm und diejenigen von Protein im Beispiel über 2200 nm liegen.

Je nach Ausbaugrad der Messeinrichtung können die Werte für Wasser und Protein beziehungsweise der Wassergehalt und der Proteingehalt gleichzeitig oder zeitlich nacheinander festgestellt werden, zum Beispiel durch Wahl entsprechender Filter.

Mit der Figur 4 soll zum Ausdruck gebracht werden, dass auch die Untersuchungen mit der neuen Erfindung die Erfahrung bestätigt hat, dass Messwerte von einem willkürlich geschütteten Haufen keine repräsentativen Werte für das Produkt an sich geben, da jede Schüttungsänderung einen Messfehler ergibt.

In Figur 5 ist das in der EP-PS Nr. 179 108 gelehrte Verfahren zur kontrollierten Verdichtung der Messprobe dargestellt. Diese Methode lässt sich mit Erfolg nur mit mehlartigen Gütern anwenden.

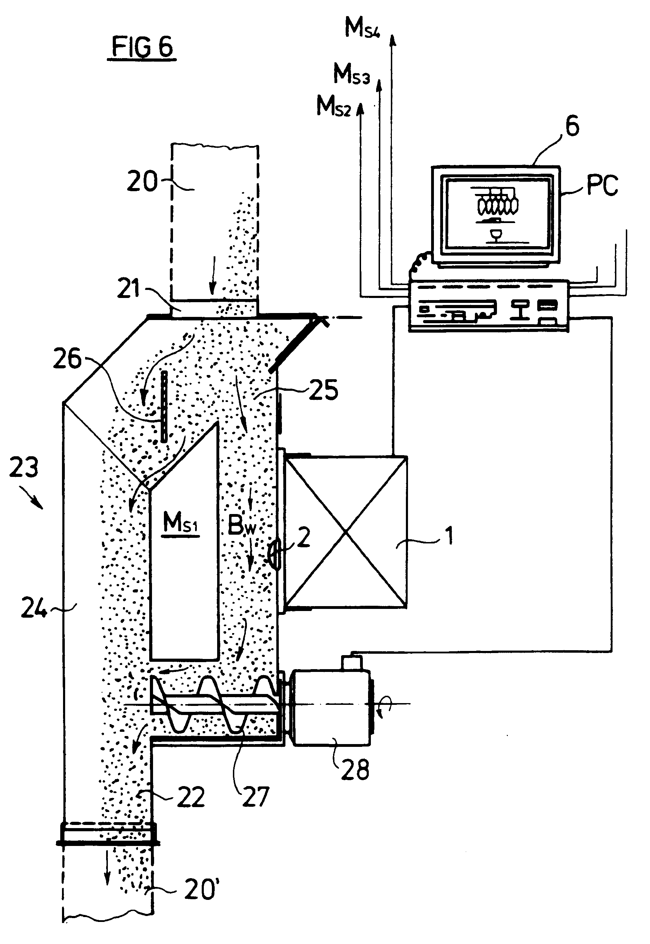

In der Figur 6 ist eine ganze Messstrecke dargestellt, welche Teil einer Produktionsförderleitung 20 resp. 20' ist, und weist oben einen Produktzuführstutzen 21 und unten einen Produktabführstutzen 22 auf.Die Messstrecke 23 ist in eine Hauptförderleitung 24 sowie eine By-Passleitung 25 aufgeteilt. Der Hauptförderstrang 24 ist im wesentlichen querschnittsgleich wie die Produktförderleitung 20 resp 20', so dass bei grösstmöglichem Durchsatz alles Produkt durch die Hauptförderleitung 24 durchströmen kann. Damit nun aber zwangsweise ein Teil des Produktstromes über den By-Pass läuft, ist im Übergangsbereich Hauptförderleitung 24 - By-Pass 25 ein Staublech 26 angeordnet.

Die Förderung des dichten Produktstromes in dem Bereich des Messkopfes 2 wird durch eine gesteuerte Austragsschnecke 27 sichergestellt. Die Austragsschnecke sorgt gleichzeitig durch Wahl einer ganz bestimmten Drehzahl für ein konstantes Absenken des Produktes in dem By-Pass respektiv in dem Messkanal 25. Dadurch erhält man eine steuerbare bzw. vorwählbare Geschwindigkeit Bw des Produktes, so dass die Produkt-Bewegung an den spezifischen Fall anpassbar ist. Interessant an der Messstrecke ist, dass entgegen der ursprünglichen Annahmen diese Lösung ganz besonders gute Resultate bei an sich rieselfähigen Schüttgütern wie ganzen Getreidekörnern ergibt, dies nachdem diese Lösung anfänglich für mehlige Güter konzipiert war, zur zwangsweisen Rückförderung des Messgutes in den Haupfförderstang 24. Die Austragsschnecke wird über einen vorzugsweise Stufenlosregelbaren Antriebsmotor 28 von der Rechnereinheit 6 gesteuert.

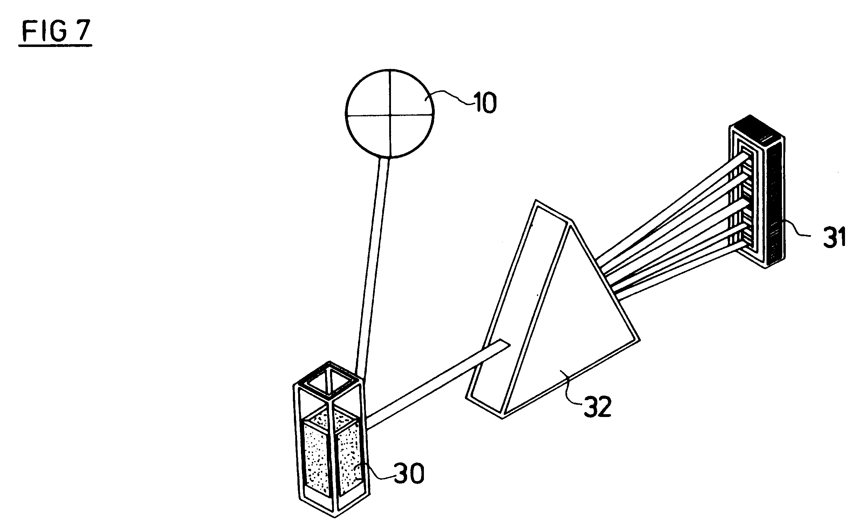

Die Figur 7 zeigt die Anwendung des Dioden-array-Prinzipes, wobei die Vorrichtung einen Probenkanal 30 eine Lichtquelle 31 sowie ein Dioden-array-Detektor 31 aufweist. Beim Dioden-array-Prinzip kann man alle gewünschten Wellenlängen gleichzeitig messen. Das zu analysierende Licht wird mit einem Polichromator 32 resp. einem Dispersionselement örtlich spektral zerlegt und auf den Dioden-array-Detektor 31 abgeleitet. Dabei lässt sich mit der Anzahl photosensitiven Dioden die Wellenlängenauflösung wählen. Der Polychromator 32 (zum Beispiel ein holographisches Gitter) kann mit dem Dioden-array-Detektor zusammengekapselt werden. Der grosse Vorteil dieses Prinzipes ist der, dass keine bewegten Teile benötigt werden, ausser eventuell ein Spiegel zur Einlenkung eines Referenzstrahles.

Bei allen Ausführungsvarianten kann einer NIR-Messaufnahmeeinrichtung eine vollständige Rechnereinheit zugeordnet werden, oder es können aber auch mehrere NIR-Messaufnahmeeinrichtungen einer gemeinsamen Rechnereinheit zugeordnet werden.

Je nach besonderen Anwendungsfällen kann es vorteilhaft sein, eine Sekunde, 10 Sekunden- oder Minuten-Mittelwerte für die Inhaltsstoffe zu ermitteln. Insbesondere für Regelaufgaben bringt der Sekundenmittelwert grosse Vorteile, wohingegen für Kontrollzwecke eine Mittelung über längere Messdauer vorteilhaft ist.

## Patentansprüche

1. Verfahren zum kontinuierlichen "in-line" NIR-Messen von Inhaltsstoffen von Schüttgütern, wie von schüttfähigen Nahrungsmittelprodukten, dadurch gekennzeichnet, dass
- das Produkt als dichter Strom kontinuierlich vertikal vor einem NIR-Messwertaufnehmer geführt wird,
- wobei der, bzw. die Wellenlängenbereiche des reflektierten Lichtes aus mehreren zeitlich nacheinander durchgeführten Einzelmessungen für je ein ganzes Spektrum von einem bewegten Probenbereich, mit Messzeiten für ein Spektrum unter 100 Millisekunden, erfasst wird
- und wobei die Messzeit einer Einzelmessung und die Bewegungsgeschwindigkeit des Produktes aufeinander abgestimmt werden, derart, dass das Produkt sich während der Zeit einer Einzelmessung um weniger als 1/10 der Länge des Produktes bewegt.
- und dass Inhaltsstoffe über statistische Mittelwertbildung der relevanten Messwerte ermittelt und aufgrund eines Eichwertes errechnet werden.

2. Verfahren nach Patentanspruch 1, dadurch gekennzeichnet, dass das Produkt aus ganzen Körnern besteht.

3. Verfahren nach einem der Patentansprüche 1 bis 3, dadurch gekennzeichnet, dass alle gewünschten Wellenlängen gleichzeitig mittels einer Diodenarray-Anordnung gemessen werden.

4. Verfahren nach Patentanspruch 1, dadurch gekennzeichnet, dass die Bewegung des Produktes vor dem NIR-Messwertaufnehmer gesteuert bzw. konstant gehalten wird, vorzugsweise durch eine Förderschnecke.

5. Verfahren nach einem der Patentansprüche 1 bis 3, dadurch gekennzeichnet, dass kurzfristig nacheinander einzelne spezifische Wellenlängen gemessen werden und eine statistische Auswertung der korrelierenden Messwerte vorgenommen wird.

6. Verfahren nach einem der Patentansprüche 1 bis 5, dadurch gekennzeichnet, dass ein kontinuierlicher Produktstrom druckfrei gestaut und das gestaute Produkt gegenüber dem Messwertaufnehmer senkrecht mit vorwählbarer Geschwindigkeit bewegt wird.

7. Verfahren nach einem der Patentansprüche 1 bis 6, dadurch gekennzeichnet, dass der Stau in einer Haupt- oder By-Passleitung durch Selbstregelung des Durchflusses erfolgt.

8. Verfahren nach einem der Patentansprüche 6 oder 7, dadurch gekennzeichnet, dass der Stau gleichzeitig zur Erfassung des Produktdurchsatzes ausgenutzt wird.

9. Verfahren nach Anspruch 1, dadurch gekennzeichnet, dass die Messzeit für ein Spektrum weniger als 50ms beträgt.

10. Messvorrichtung zum kontinuierlichen in line NIR-Messen von Inhaltsstoffen von Schüttgütern, wie an einem schüttfähigen Nahrungsmittel, die einer NIR-Messeinrichtung zur Durchführung des Verfahrens nach Anspruch 1, dadurch gekennzeichnet, dass sie eine vorzugsweise rohrförmige Durchlaufmessstrecke, eine NIR-Messaufnahme-Einrichtung, für viele Messungen und jeweiliger Messdauer unter 100 Millisekunden pro Einzelmessung und ein Dioden-array für die gleichzeitige Erfassung einzelner Spektralbereiche, sowie einen Rechner für die statistische Ermittlung von Mittelwerten der jeweiligen Inhaltsstoffe aufweist, sowie einen Messkanal mit Mitteln zur gesteuerten Austragszwangsbewegung der Messprobe gegenüber der NIR-Messaufnahme-Einrichtung aufweist.

11. Messvorrichtung nach Patentanspruch 10, dadurch gekennzeichnet, dass sie ein Umlauffilterprinzip mit Dauerrotation von mehr als 10 Umdrehungen pro Sekunde zur zeitlich nacheinander Erfassung einzelner Spektralbereiche mit der Auswahl relevanter Wellenlängen bzgl. der Inhaltsstoffe über entsprechende Filter, sowie Detektoren aufweist.

## Claims

1. Process for the continuous "in-line" NIR measuring of constituents of bulk materials, such as pourable food products, characterised in that
- the product is conducted as a dense flow continuously vertically in front of an NIR measurement value sensor,
- wherein the wavelength range or ranges of the reflected light is/are ascertained from a plurality of individual measuring operations, carried out in time succession, each for a whole spectrum of a moved sample range, with measuring times for a spectrum below 100 milliseconds,
- and wherein the measuring time of an individual measuring operation and the speed of movement of the product are adapted to one another in such a way that, during the time of an individual measuring operation, the product moves by less than 1/10 of the length of the product,
- and in that the constituents are determined by statistical averaging of the relevant measurement values and calculated on the basis of a calibration value.

2. Process according to claim 1, characterised in that the product consists of whole grains.

3. Process according to one of claims 1 to 3, characterised in that all the desired wavelengths are measured simultaneously by means of a diode array arrangement.

4. Process according to claim 1, characterised in that the movement of the product in front of the NIR measurement value sensor is controlled or kept constant, preferably by a conveying worm.

5. Process according to one of claims 1 to 3, characterised in that individual specific wavelengths are measured in quick succession, and statistical evaluation of the correlating measurement values is effected.

6. Process according to one of claims 1 to 5, characterised in that a continuous product flow is dammed-up without the use of pressure, and the dammed-up product is moved relative to the measurement value sensor vertically at a preselectable speed.

7. Process according to one of claims 1 to 6, characterised in that the damming-up occurs in a main or a bypass conduit as a result of self-regulation of the throughflow.

8. Process according to one of claims 6 or 7, characterised in that the damming-up is utilised at the same time for ascertaining the product throughput.

9. Process according to claim 1, characterised in that the measuring time for a spectrum amounts to less than 50 ms.

10. Measuring apparatus for the continuous in-line NIR measuring of constituents of bulk materials, such as a pourable foodstuff, with an NIR measuring device for carrying out the process according to claim 1, characterised in that it comprises a preferably tubular throughflow measuring section, an NIR measurement sensor device, for many measuring operations and a respective measuring duration of less than 100 milliseconds per individual measuring operation, and a diode array for the simultaneous detection of individual spectral ranges, also a computer for the statistical determining of mean values of the respective constituents, and also comprises a measuring duct with means for the controlled forcible discharge movement of the sample being measured relative to the NIR measurement sensor device.

11. Measuring apparatus according to claim 10, characterised in that it involves a rotary filter principle with continuous rotation of more than 10 revolutions per second for the detecting in time succession of individual spectral ranges with the choice of relevant wavelengths, or constituents, via appropriate filters, and also detectors.

## Revendications

1. Procédé pour la mesure NIR, "en ligne" et continue, des substances contenues dans des matières en vrac, telles que des produits alimentaires déversables,
caractérisé en ce que
- le produit est envoyé, d'une manière continue et verticalement, sous forme d'un flux dense, devant un capteur de valeur mesurée NIR,
- le ou les domaines de longueur d'onde de la lumière réfléchie étant déterminés à partir de plusieurs mesures individuelles exécutées l'une à la suite de l'autre dans le temps et correspondant chacune à un spectre complet provenant d'une zone d'échantillon en mouvement, avec des durées de mesure pour un spectre qui sont inférieures à 100 millisecondes,
- et la durée de mesure d'une mesure individuelle et la vitesse de déplacement du produit sont adaptées l'une à l'autre, d'une façon telle que le produit se déplace de moins de 1/10 de la longueur du produit pendant la durée d'une mesure individuelle,
- et en ce que les substances contenues sont déterminées au moyen d'une formation de valeur moyenne statistique des valeurs mesurées pertinentes et sont calculées sur la base d'une valeur d'étalonnage.

2. Procédé selon la revendication 1, caractérisé en ce que le produit est constitué de grains entiers.

3. Procédé selon l'une des revendications 1 et 2, caractérisé en ce que toutes les longueurs d'onde voulues sont mesurées en même temps au moyen d'un agencement à réseau de diodes.

4. Procédé selon la revendication 1, caractérisé en ce que le mouvement du produit devant le capteur de valeur mesurée NIR est commandé ou maintenu constant, de préférence au moyen d'un transporteur à vis sans fin.

5. Procédé selon l'une des revendications 1 à 3, caractérisé en ce que des longueurs d'onde spécifiques individuelles sont mesurées à un court intervalle de temps à la suite l'une de l'autre et une analyse statistique des valeurs mesurées en corrélation est effectuée.

6. Procédé selon l'une des revendications 1 à 5, caractérisé en ce qu'un flux continu de produit fait l'objet d'une retenue sans pression et le produit retenu est déplacé perpendiculairement vis-à-vis du capteur de valeur de mesure à une vitesse pouvant être choisie à l'avance.

7. Procédé selon l'une des revendications 1 à 6, caractérisé en ce que la retenue a lieu dans une conduite principale ou une conduite de déviation par autorégulation du débit.

8. Procédé selon l'une des revendications 6 ou 7, caractérisé en ce que la retenue est utilisée en même temps pour déterminer le débit de produit.

9. Procédé selon la revendication 1, caractérisé en ce que la durée de mesure pour un spectre a une valeur inférieure à 50 ms.

10. Dispositif de mesure pour la mesure NIR, en ligne et continue, des substances contenues dans des matières en vrac, par exemple sur un produit alimentaire déversable, au moyen d'un dispositif de mesure NIR et pour la mise en oeuvre du procédé selon la revendication 1, caractérisé en ce qu'il comprend une section de mesure en continu, de préférence tubulaire, un dispositif de prise de mesure NIR, pour de nombreuses mesures et avec chaque fois une durée de mesure inférieure à 100 millisecondes par mesure individuelle, un réseau de diodes pour la saisie simultanée de domaines spectraux individuels et un ordinateur pour la détermination statistique de valeurs moyennes des substances contenues respectives, ainsi qu'un conduit de mesure comportant des moyens pour un déplacement forcé d'extraction, commandé, de l'échantillon de mesure vis-à-vis du dispositif de prise de mesure NIR.

11. Dispositif de mesure selon la revendication 10, caractérisé en ce qu'il comprend un système de filtre à mouvement de révolution présentant une rotation continue à plus de 10 tours par seconde et servant à la saisie de domaines spectraux individuels l'un à la suite de l'autre dans le temps, avec le choix de longueurs d'onde pertinentes en ce qui concerne les substances contenues, au moyen de filtres appropriés, ainsi que des capteurs.
